(19) Europäisches Patentamt / European Patent Office / Office européen des brevets

(11) **EP 4 524 991 A1**

(12) **EUROPEAN PATENT APPLICATION**
published in accordance with Art. 153(4) EPC

(43) Date of publication:
**19.03.2025 Bulletin 2025/12**

(51) International Patent Classification (IPC):
*G16H 50/30* (2018.01)    *G16H 50/50* (2018.01)
*G16H 50/70* (2018.01)    *G16B 40/00* (2019.01)

(21) Application number: **22941242.4**

(86) International application number:
**PCT/CN2022/093822**

(22) Date of filing: **19.05.2022**

(87) International publication number:
**WO 2023/216293 (16.11.2023 Gazette 2023/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **11.05.2022 CN 202210510090**

(71) Applicant: **Hangzhou Qingguo Medical
Technology Co., Ltd.
Hangzhou, Zhejiang 310000 (CN)**

(72) Inventors:
• **HAN, Yong**
**Hangzhou, Zhejiang 310000 (CN)**
• **FENG, Guoshuang**
**Hangzhou, Zhejiang 310000 (CN)**
• **XU, Huiyu**
**Beijing 100191 (CN)**
• **ZHANG, Hongxian**
**Beijing 100191 (CN)**
• **ZHANG, Li**
**Hangzhou, Zhejiang 310000 (CN)**

(74) Representative: **Keller Schneider
Patentanwaltsgesellschaft mbH
Linprunstraße 10
80335 München (DE)**

(54) **SYSTEM AND METHOD FOR PREDICTING DEMENTIA OR MILD COGNITIVE DISORDER**

(57)    A system for predicting dementia or mild cognitive disorder, which system comprises: a data acquisition module, which is used for acquiring the miRNA level, apolipoprotein E4 genotype, age and gender data of a subject; and a module for calculating the probability of developing dementia or mild cognitive disorder (MCI), wherein the module is used for calculating the probability (p) of a subject developing dementia or MCI using the data obtained from the data acquisition module. On one hand, the system can be used as a tool for diagnosing MCI and other dementias, and on the other hand, the system can be used as a potential drug target for MCI, thereby preventing or delaying the progression of MCI.

| | Training set (n=838) | Validation set (n=209) | Test set (n=262) |
|---|---|---|---|
| AUC | 0.878 (0.856 - 0.900) | 0.875 (0.830 - 0.920) | 0.874 (0.834 - 0.914) |
| True positive rate | 0.953 (0.933 - 0.966) | 0.963 (0.922 - 0.983) | 0.917 (0.871 - 0.948) |
| True negative rate | 0.556 (0.485 - 0.626) | 0.565 (0.422 - 0.698) | 0.579 (0.450 - 0.698) |

Fig. 1

**Description**

**TECHNICAL FIELD**

The present application relates to a system and method for predicting dementia or mild cognitive disorder.

**BACKGROUND**

Dementia is a major neurocognitive disorder that affects memory, thinking, language and behavior, all of which can interfere with daily life. The World Alzheimer's Report 2010 estimates that the aging of the global population will make the economic impact of dementia greater than a combination of cancer, heart disease and stroke. It is estimated that there will be 75 million people worldwide by 2030, and 135 million by 2050, placing a huge burden on healthcare and public health systems. Currently, treatments are only used to manage symptoms and slow the progression of dementia, since there is no known cure. At the same time, as China's birth rate continues to decline, the country is facing an increasingly serious situation that may be brought about by dementia, namely, there will be fewer and fewer working-age adults who can provide continuous care for millions of dementia patients. Therefore, early diagnosis and therapeutic intervention of dementia are very important.

The diagnosis of dementia (major neurocognitive disorder) and mild cognitive disorder (mild neurocognitive disorder/MCI) is based on medical history, examination, assessment of cognitive function, brain imaging, and cerebrospinal fluid (CSF) biomarkers. Alzheimer's disease (AD) is the most common type of dementia, followed by vascular dementia (VaD) and dementia with Lewy bodies (DLB). Although patients suffering the dementia cannot be cured at this time, it is treatable if it is detected early. To identify these patients, cognitive assessment is currently a most convenient and commonly used method. Currently, biomarkers in cerebrospinal fluid are the most reliable indicators for diagnosing AD, including three core CSF biomarkers: amyloid-$\beta$ (A$\beta$), total tau, and phosphorylated tau. However, CSF markers are highly invasive, and are only useful when clinical cognitive disorder is present. Furthermore, although some new imaging-based technologies are gaining attention, they are not cost-effective, and are not suitable for early screening of Alzheimer's disease. Ultimately, the ideal AD biomarker would be noninvasive, easy to use, cost-effective, and identify early signs of the neurodegenerative process before clinical appearance of cognitive abnormalities. Currently, there is no existing non-invasive model that can simultaneously screen for the three main types of dementia and mild cognitive disorder (MCI).

**SUMMARY**

The object of the present application is to provide an effective system for predicting dementia, which comprises the three most common types of dementia (AD, VaD and DLB), and can also be used for predicting MCI.

In summary, the present application relates to the following contents:

> 1. A system for predicting dementia or mild cognitive disorder, comprising:
>
>> a data acquisition module for acquiring data on the miRNA level, apolipoprotein E4 genotype, age and gender of a subject; and
>> a module for calculating the probability of suffering from dementia or mild cognitive disorder, which is used to calculate the data obtained in the data acquisition module, thereby determining the probability (p) of the subject suffering from dementia or mild cognitive disorder.
>
> 2. The system according to item 1, wherein the miRNA is one or more selected from the group consisting of: hsa-miR-6761-3p, hsa-miR-3173-5p and hsa-miR-6716-3p; preferably the miRNA comprises hsa-miR-6761-3p, hsa-miR-3173-5p and hsa-miR-6716-3p.
> 3. The system according to item 1, wherein the apolipoprotein E4 genotype of the subject refers to the typing of the apolipoprotein E4 allele.
> 4. The system according to item 1, wherein in the module for calculating the probability of suffering from dementia or mild cognitive disorder, a formula for calculating the probability (p) of suffering from dementia is pre-stored, and the formula is obtained by fitting through logistic regression on the basis of the data on the miRNA level, apolipoprotein E4 genotype, age and gender of subjects in an existing database.
> 5. The system according to item 4, wherein the formula is the following Formula 1:

$$p=1/[1+e^{-(i+a*Age+b*Gender+c*APOE\ Typing+d*hsa\text{-}miR\text{-}6761\text{-}3p+f*hsa\text{-}miR\text{-}3173\text{-}5p+g*hsa\text{-}miR\text{-}6716\text{-}3p)}]$$

(Formula 1);

> wherein p is the probability of suffering from dementia or mild cognitive disorder; APOE typing is the apolipoprotein E4 genotype of the subject, and i, a, b, c, d, f and g are unitless parameters;
> in the module for calculating the probability of suffering from dementia, the values of a, b, c, d, f and g are obtained on the basis of the miRNA level, apolipoprotein E4 genotype, age and gender of the subject, and the values are substituted into Formula 1 for calculation.

6. The system according to item 5, wherein

> i is any value selected from -22.77226 to -15.70663, preferably -19.23944;
> a is any value selected from 0.1653123 to 0.2425499, preferably 0.2039311;
> d is any value selected from 0.8932064 to 1.7053113, preferably 1.2992589;
> f is any value selected from -0.722253 to -0.073824, preferably -0.398038; and
> g is any value selected from 0.0263939 to 0.5324189, preferably 0.2794064.

7. The system according to item 5, wherein

> when the subject is a female, b takes the value of 0; and
> when the subject is a male, b is any value selected from -1.144378 to -0.309418, preferably -0.726898.

8. The system according to item 5, wherein

> when the subject does not express the APOE4 genotype, c takes the value of 0;
> when the subject's APOE4 genotype is homozygous, c takes any value selected from 1.1429478 to 3.7701044, preferably 2.4565261; and
> when the subject's APOE4 genotype is heterozygous, c takes any value selected from 0.9740697 to 2.038065, preferably 1.5060673.

9. The system according to item 1, further comprising:
a grouping module, in which default grouping parameters for dementia or mild cognitive disorder are pre-stored, and the calculated probability (p) of the subject suffering from dementia or mild cognitive disorder is grouped on the basis of the grouping parameters, thereby grouping the risk of the subject suffering from dementia or mild cognitive disorder.
10. The system according to item 9, wherein the grouping basis pre-stored in the grouping module is:

> when the calculated probability (p) of a subject suffering from dementia or mild cognitive disorder is less than 10%, the risk of the subject suffering from dementia or mild cognitive disorder is low;
> when the calculated probability (p) of the subject suffering from dementia or mild cognitive disorder is equal to or greater than 10% and less than 50%, the risk of the subject suffering from dementia or mild cognitive disorder is medium;
> when the calculated probability (p) of the subject suffering from dementia or mild cognitive disorder is equal to or greater than 50% and less than 90%, the risk of the subject suffering from dementia or mild cognitive disorder is medium-high; and
> when the calculated probability (p) of the subject suffering from dementia or mild cognitive disorder is equal to or greater than 90%, the risk of the subject suffering from dementia or mild cognitive disorder is high.

11. A method for predicting dementia or mild cognitive disorder, comprising:

> a data acquisition step, which obtains data on the miRNA level, apolipoprotein E4 genotype, age and gender of a subject; and
> a step for calculating the probability of suffering from dementia or mild cognitive disorder, which is used to calculate the data acquired in the data acquisition step, thereby determining the probability (p) of the subject suffering from dementia or mild cognitive disorder.

12. The method according to item 11, wherein the miRNA is one or more selected from the group consisting of: hsa-miR-6761-3p, hsa-miR-3173-5p and hsa-miR-6716-3p; preferably the miRNA comprises hsa-miR-6761-3p, hsa-miR-3173-5p and hsa-miR-6716-3p.

13. The method according to item 11, wherein the apolipoprotein E4 genotype of the subject refers to the typing of the apolipoprotein E4 allele.

14. The method according to item 11, wherein in the step for calculating the probability of suffering from dementia or mild cognitive disorder, a formula for calculating the probability (p) of suffering from dementia is pre-stored, and the formula is obtained by fitting through logistic regression on the basis of the data on the miRNA level, apolipoprotein E4 genotype, age and gender of subjects in an existing database.

15. The method according to item 14, wherein the formula is the following Formula 1:

$$p = 1/[1 + e^{-(i + a*Age + b*Gender + c*APOE\ Typing + d*hsa\text{-}miR\text{-}6761\text{-}3p + f*hsa\text{-}miR\text{-}3173\text{-}5p + g*hsa\text{-}miR\text{-}6716\text{-}3p)}]$$

(Formula 1);

wherein p is the probability of suffering from dementia or mild cognitive disorder; APOE typing is the apolipoprotein E4 genotype of the subject, and i, a, b, c, d, f and g are unitless parameters;
in the step for calculating the probability of suffering from dementia, the values of a, b, c, d, f and g are obtained on the basis of the miRNA level, apolipoprotein E4 genotype, age and gender of the subject, and the values are substituted into Formula 1 for calculation.

16. The method according to item 15, wherein

i is any value selected from -22.77226 to -15.70663, preferably -19.23944;
a is any value selected from 0.1653123 to 0.2425499, preferably 0.2039311;
d is any value selected from 0.8932064 to 1.7053113, preferably 1.2992589;
f is any value selected from -0.722253 to -0.073824, preferably -0.398038; and
g is any value selected from 0.0263939 to 0.5324189, preferably 0.2794064.

17. The method according to item 15, wherein

when the subject is a female, b takes the value of 0; and
when the subject is a male, b is any value selected from -1.144378 to -0.309418, preferably -0.726898.

18. The method according to item 15, wherein

when the subject does not express the APOE4 genotype, c takes the value of 0;
when the subject's APOE4 genotype is homozygous, c takes any value selected from 1.1429478 to 3.7701044, preferably 2.4565261; and
when the subject's APOE4 genotype is heterozygous, c takes any value selected from 0.9740697 to 2.038065, preferably 1.5060673.

19. The method according to item 11, further comprising:
a grouping step, in which default grouping parameters for dementia or mild cognitive disorder are pre-stored, and the calculated probability (p) of the subject suffering from dementia or mild cognitive disorder is grouped on the basis of the grouping parameters, thereby grouping the risk of the subject suffering from dementia or mild cognitive disorder.

20. The method according to item 19, wherein a grouping basis pre-stored in the grouping step is:

when the calculated probability (p) of the subject suffering from dementia or mild cognitive disorder is less than 10%, the risk of the subject suffering from dementia or mild cognitive disorder is low;
when the calculated probability (p) of the subject suffering from dementia or mild cognitive disorder is equal to or greater than 10% and less than 50%, the risk of the subject suffering from dementia or mild cognitive disorder is medium;
when the calculated probability (p) of the subject suffering from dementia or mild cognitive disorder is equal to or greater than 50% and less than 90%, the risk of the subject suffering from dementia or mild cognitive disorder is medium-high; and
when the calculated probability (p) of the subject suffering from dementia or mild cognitive disorder is equal to or greater than 90%, the risk of the subject suffering from dementia or mild cognitive disorder is high.

Effects of the Application

The present application establishes a mathematical model for predicting whether a subject suffers from dementia or mild cognitive disorder based on the subject's miRNA level, the subject's apolipoprotein E4 genotype, the subject's age, and the subject's gender. The parameters used in the system established in this application can all be detected simply and non-invasively. The system of the present application is a non-invasive system for predicting the three main types of dementia, and is suitable for predicting MCI. The model has excellent universality, and can be used to conduct universal screening of the population to identify as many people as possible who are at potential risk. It is of great significance for non-predictive MCI. The system of the present application can be used as a tool for diagnosing MCI and other dementias on one hand, and as a potential drug target for MCI on the other hand, thereby preventing or slowing the progression of MCI.

## BRIEF DESCRIPTION OF THE DRAWINGS

Various other advantages and benefits of the present application will become apparent to those of ordinary skill in the art by reading the following detailed description of the preferred particular embodiments. The drawings accompanying the specification are only used for illustrating the preferred embodiments, and should not to be considered as limiting the present application. Obviously, the drawings described below are only some embodiments of the present application. For a person skilled in the art, other drawings can be obtained based on these drawings without any creative work. Also, throughout the drawings, the same reference numerals are used to denote the same components.

Fig. 1 shows the predicted results of the prediction model in the training set, test set and validation set of AD.
Fig. 2 shows the predicted results of the prediction model in the training set, test set, and validation set of VAD, DLB, and MCI.
Fig. 3 shows the grouping results of the predicted probability and actual incidence of the prediction model in AD data.

## DETAILED DESCRIPTION

Particular Examples of the present application will be described in more detail below with reference to the accompanying drawings. Although particular examples of the present application are shown in the drawings, it should be understood that the application can be embodied in various forms, and should not be limited to the examples set forth herein. On the contrary, these examples are provided to enable a more thorough understanding of the present application, and to fully convey the scope of the present application to those skilled in the art.

It should be noted that, certain terms are used in the specification and claims to refer to specific components. Those skilled in the art should understand that, they may use different terms to refer to the same component. In this specification and claims, differences in nouns are not used as a way to distinguish components, while differences in functions of components are used as the criterion for distinction. As mentioned throughout the specification and claims, "comprise/comprising" or "include/including" is an open-ended term, and should be interpreted as "including but not limited to". The following description are preferred embodiments of the present application, but such description is for the purpose of general principles of description, and is not intended to limit the scope of the present application. The protection scope of the present application shall be determined by the appended claims.

Variable type: in statistics, variable types can be divided into quantitative variables and qualitative variables (also called categorical variables).

Quantitative variables are variables used to describe the quantity and number of things, and can be divided into continuous and discrete types. A continuous variable is a variable that can take any value within a certain interval, and its value is continuous and can have decimal points. For example, blood pressure values, blood sugar values, human height, weight, chest circumference, etc. are continuous variables, and their values can only be obtained by measurement or metering methods. A discrete variable is a variable whose value can only be a natural number or an integer unit. For example, pain scores, the number of lesion metastases, the number of eggs retrieved, etc., can only be positive numbers and cannot have decimal points; the values of such variables are generally obtained by counting methods.

The variable type is not static. Different types of variables can be transformed according to the needs of the research purpose. For example, the hemoglobin level (g/L) is originally a numerical variable. If it is divided into two categories, normal and low hemoglobin, it can be analyzed according to binomial classification data. If it is divided into five levels: severe anemia, moderate anemia, mild anemia, normal, and increased hemoglobin, it can be analyzed according to level data. Sometimes categorical data can also be quantified. For example, the patient's nausea reaction can be represented by 0, 1, 2, or 3, and then analyzed according to numerical variable data (quantitative data).

Logistic regression is a generalized linear regression analysis model, which is often used in data mining, automatic disease diagnosis, economic forecasting and other fields, for example, exploring the risk factors that cause disease, and predicting the probability of disease occurrence based on the risk factors. Taking the analysis of gastric cancer as an example, two groups of people are selected, one is the gastric cancer group, and the other is the non-gastric cancer group. The two groups of people must have different physical signs and lifestyles. Therefore, the dependent variable is whether or not there is gastric cancer, with a value of "yes" or "no", and the independent variables can include many factors, such as age, gender, eating habits, *Helicobacter pylori* infection, etc. Independent variables can be either continuous or categorical. Then, through logistic regression analysis, the weights of the independent variables can be obtained, so that we can roughly understand which factors are the risk factors for gastric cancer. At the same time, the weight can be used to predict the possibility of a person suffering from cancer based on risk factors. The dependent variable of logistic regression can be binary or multi-class.

The data used in this application are fitted into a logistic regression model by logistic regression, which penalizes the absolute size of the coefficients of the regression model based on the value of $\lambda$. The larger the penalty, the closer the estimates of weaker factors are to zero, so only the strongest predictors remain in the model.

MicroRNA (miRNA) is an endogenous noncoding RNA with approximately 22 nucleotides, which can regulate gene expression at the post-transcriptional level. Due to its small molecular weight, it can break away from cell membranes, and travel through the blood circulation. Therefore, miRNA can be used as a powerful tool for non-invasive screening of diseases. Accumulating evidence indicates that microRNAs play a key role in different pathological processes throughout AD progression. In this application, the inventors attempt to use miRNA and other basic clinical information to establish a non-invasive model for early identification of dementia, thereby facilitating early intervention of dementia-related symptoms.

In this application, serum miRNAs mainly come from microvesicle-mediated active secretion. They exhibit remarkable stability in the extracellular environment for a long time, mainly due to their interaction with Argonaute2-miRNA complexes or lipoprotein complexes or vesicles. Therefore, miRNAs, as key regulators of gene expression, are increasingly being recognized as promising candidates for novel non-invasive, inexpensive, and sensitive diagnostic biomarkers. Differently expressed serum miRNAs, such as miR-31, -93, -143, -146a, -135a, -193b, and -384, have been previously found in AD patients by using relatively the same sample amounts. Since the data sources studied by the applicant of this application have made significant progress, prospective cohort data of large samples were used to establish a single model to predict different dementia subtypes and mild cognitive disorder.

The data source team of this application used serum miRNA expression data from 1,601 Japanese, and established three different models by using data from AD, VaD, DLB and normal control populations, respectively. The AUCs in the AD, VaD and DLB models are 0.874, 0.867 and 0.870, respectively, and the numbers of miRNAs discovered by the three prediction models are 78 miRNAs, 86 miRNAs and 110 miRNAs, respectively. The authors of the data source team of this application established three different models based on three different dementia data, and the models have quite a lot of independent variables. On one hand, this generally means smaller practical significance in statistics and practical experience. On the other hand, different predictive variables are used in different models, and no common rules are found for different dementias, suggesting that the models have lower application value. AD dementia as the most common type of dementia was used in this application to construct a model, three key miRNAs were found, and the model was verified with internal and external data, suggesting better stability of the model. In addition, the established model is not only effective in AD dementia, but also has good predictive effects in different types of dementia and MCI, suggesting that the three miRNAs discovered by the model of this application may have discovered common rules for different types of dementia and mild cognitive disorder, and therefore have more important significance. In the future, it may be used for the diagnosis and screening of dementia and mild cognitive disorder, and on the other hand, it may even be used as a target for drug development. It has potentially important social and economic significance.

The greatest risk factor for neurodegenerative diseases is advanced age. APOE epsilon4 (apolipoprotein E4, APOE4) genotype and female gender are two well-known risk factors for AD as age increases. It is speculated that menopause and ovarian estrogen deficiency are responsible for the increased incidence of AD in women over 65 years old. Estrogen is the major female sex hormone, and plays important roles in both reproductive and non-reproductive systems, including neuroprotection. Estrogen therapy initiated early in menopause, when neurons are healthy, has beneficial effects on neuroprotection. Patterns of APOE epsilon4 allele status were also found to be associated with estrogen treatment. In addition, subjects with the APOE epsilon4 genotype had a 15-fold increased risk as compared with the normal genotype. APOE epsilon4 also contributes to the progression of atherosclerosis and neurodegenerative diseases.

In order to solve the problems existing in the prior art, the present application relates to a system for predicting dementia or mild cognitive disorder, which comprises: a data acquisition module for acquiring data on the miRNA level, apolipoprotein E4 genotype, age and gender of a subject; and a module for calculating the probability of suffering from dementia or mild cognitive disorder, which is used to calculate the data obtained in the data acquisition module, thereby determining the probability (p) of the subject suffering from dementia or mild cognitive disorder.

In this application, there is no limitation on the data acquisition module, as long as it can be used to acquire data on the subject's miRNA level, the subject's apolipoprotein E4 (APOE4) genotype, the subject's age, and the subject's gender.

Particularly, the miRNA level of the subject obtained by the data acquisition module refers to the abundance of miRNA in the serum, which can be detected by existing sequencing, PCR or miRNA chip methods, etc.

When there are multiple miRNAs, the data acquisition module obtains the expression level of each miRNA respectively. The miRNA level of the subject can be obtained through existing chip detection.

In a particular embodiment, the miRNA is one or more selected from the group consisting of: hsa-miR-6761-3p, hsa-miR-3173-5p, and hsa-miR-6716-3p.

In a particular embodiment, the miRNA comprises hsa-miR-6761-3p, hsa-miR-3173-5p, and hsa-miR-6716-3p. At this time, the data acquisition module needs to obtain the levels of the subjects' three miRNAs of hsa-miR-6761-3p, hsa-miR-3173-5p and hsa-miR-6716-3p, respectively.

A large number of miRNAs were preliminarily screened in this application, but in-depth research by the applicant showed that, the most significant effect can be obtained when one, two or three of the three miRNAs of hsa-miR-6761-3p, hsa-miR-3173-5p and hsa-miR-6716-3p, are selected, and the method and system of this application can be used to predict the three main types of dementia and mild cognitive disorder (MCI). Although there have been studies in the prior art on predicting by using different miRNAs, a universal system and method for preliminary screening of Alzheimer's disease has not yet been established in this field. This application has finally selected 3 miRNAs from the 2562 miRNAs in the miRNA chip after a large number of experiments, and constructed the method and system of this application. The three main types of dementia are AD, VaD dementia, and DLB dementia. Mild cognitive disorder refers to a disease state between normal aging and dementia. Compared with normal elderly people matched in age and education level, the patients have mild cognitive disorder, but their daily abilities are not significantly affected. The core symptom of mild cognitive disorder is the decline of cognitive function. Depending on different causes or locations of brain damage, it may affect one or more of memory, executive function, language, application, visual-spatial structural skills, etc., leading to corresponding clinical symptoms. In the prior art, there has been a lack of methods and systems for extensive and universal screening of the three main types of Alzheimer's disease and mild cognitive disorder, and the method and system according to the present application fills this gap.

In the present application, the module for calculating the probability of suffering from dementia or mild cognitive disorder is used to calculate the above data acquired in the data acquisition module, thereby calculating the probability (p) of the subject suffering from dementia or mild cognitive disorder. Firstly, it should be understood that, the module pre-stores a formula for calculating the probability (p) of suffering from dementia based on the data of the subject's miRNA level, the subject's apolipoprotein E4 genotype, the subject's age and the subject's gender in an existing database by fitting through logistic regression. With this pre-stored formula, calculations can be performed for any subject.

In the present application, the existing database refers to a database of subjects that can be obtained. There is no agreement on the sample size of the database. Of course, the larger the sample size of the database, the better, for example, it may be 100 subjects, 200 subjects, 300 subjects, preferably more than 400 subjects, and more preferably more than 500 subjects.

During calculation, the pre-stored formula uses the data of the subject's miRNA level, the subject's apolipoprotein E4 genotype, the subject's age and the subject's gender collected by the data acquisition module, so as to calculate the probability of the subject suffering from dementia.

Particularly, the gender of the subject is a two-categorical variable, and the apolipoprotein E4 allele status of the subject is a three-categorical variable. The miRNA levels and age of the subjects are continuous variables.

Furthermore, the inventors of the present application have constructed a specific formula for predicting the probability (p) of the subject suffering from dementia, which is the following Formula 1:

$$p = 1/[1 + e^{-(i + a*Age + b*Gender + c*APOE\ Typing + d*hsa\text{-}miR\text{-}6761\text{-}3p + f*hsa\text{-}miR\text{-}3173\text{-}5p + g*hsa\text{-}miR\text{-}6716\text{-}3p)}]$$

(Formula 1);

Further, in the Formula 1, p is the probability of suffering from dementia or early cognitive disorder; APOE4 genotype is the apolipoprotein E4 allele status of the subject, and i, a, b, c, d, f and g are unitless parameters;

In the module for calculating the probability of suffering from dementia, the values of a, b, c, d, f and g are obtained based on the subject's miRNA level, the subject's apolipoprotein E4 genotype, the subject's age and the subject's gender, and the values are substituted into Formula 1 for calculation.

In a particular embodiment,

i is any value selected from -22.77226 to -15.70663, preferably -19.23944;
a is any value selected from 0.1653123 to 0.2425499, preferably 0.2039311;
d is any value selected from 0.8932064 to 1.7053113, preferably 1.2992589;
f is any value selected from -0.722253 to -0.073824, preferably -0.398038; and

g is any value selected from 0.0263939 to 0.5324189, preferably 0.2794064.

In a particular embodiment, the gender of the subject is a two-categorical variable. When the subject is female, b is 0; when the subject is male, b is any value selected from -1.144378 to -0.309418, preferably -0.726898.

In a particular embodiment, the apolipoprotein E allele status of the subject is a three-categorical variable. When the subject does not express the APOE4 genotype, c takes a value of 0; when subject's APOE4 genotype is homozygous, c takes any value from 1.1429478 to 3.7701044, preferably 2.4565261; when subject's APOE4 genotype is heterozygous, c takes any value from 0.9740697 to 2.038065, preferably 1.5060673.

Furthermore, the system of the present application may also comprise a grouping module, in which default grouping parameters for dementia or mild cognitive disorder are pre-stored, and based on these grouping parameters, the calculated probability (p) of the subject suffering from dementia or mild cognitive disorder is grouped, thereby grouping the risk of the subject suffering from dementia or mild cognitive disorder.

The grouping basis pre-stored in the grouping module is:

when the calculated probability (p) of a subject suffering from dementia or mild cognitive disorder is less than 10%, the risk of the subject suffering from dementia or mild cognitive disorder is low;

when the calculated probability (p) of the subject suffering from dementia or mild cognitive disorder is equal to or greater than 10% and less than 50%, the risk of the subject suffering from dementia or mild cognitive disorder is medium;

when the calculated probability (p) of the subject suffering from dementia or mild cognitive disorder is equal to or greater than 50% and less than 90%, the risk of the subject suffering from dementia or mild cognitive disorder is medium-high; or

when the calculated probability (p) of the subject suffering from dementia or mild cognitive disorder is equal to or greater than 90%, the risk of the subject suffering from dementia or mild cognitive disorder is high.

The present application also relates to a method for predicting dementia or mild cognitive disorder, comprising:

a data acquisition step, which obtains data on the miRNA level, apolipoprotein E4 genotype, age and gender of a subject; and

a step for calculating the probability (p) of suffering from dementia or early cognitive disorder, which is used to calculate the data acquired in the data acquisition step, thereby determining the probability (p) of the subject suffering from dementia or early cognitive disorder.

As described above, the specific contents of the data acquisition step and the step for calculating the probability of suffering from dementia or early cognitive disorder involved in the method for predicting dementia or early cognitive disorder of the present application, such as obtaining data on the miRNA level, apolipoprotein E4 genotype, age and gender of a subject, and calculating the probability (p) of the subject suffering from dementia or early cognitive disorder can refer to the above description about the various modules of the system involved in the present application.

**EXAMPLES**

**Data Used to Build the Model**

The data for constructing a model consists of 1,309 samples from 1,021 AD patients and 288 healthy controls. Particularly, 91 VaD patients, 169 DLB patients, and 32 MCI patients were further used to evaluate the performance of the AD prediction model in other types of dementia and mild cognitive disorder.

The serum miRNA chip data of the above subjects and the corresponding age, gender and APOE allele genotype were downloaded from GEO (Gene Expression Omnibus) with the storage number GSE120584.

According to GSE120584 and related articles (Shigemizu D, Akiyama S, Asanomi Y, Boroevich KA, Sharma A, Tsunoda T, Matsukuma K, Ichikawa M, Sudo H, Takizawa S *et al*: Risk prediction models for dementia constructed by supervised principal component analysis using miRNA expression data. Commun Biol 2019,2:77), all 1601 subjects were >60 years old, had APOE4 genotype detected, and underwent Mini-Mental State Exam (MMSE) assessment.

The diagnosis of all patients and healthy controls was based on medical history, physical examination, diagnostic tests, neurologic examination, neuropsychological test, and brain imaging by magnetic resonance imaging (MRI) or computed tomography (CT). Neuropsychological test includes MMSE, Alzheimer's Disease Assessment Scale Cognitive Component Japanese version, logical memory I and II from the Wechsler Memory Scale-Revised, frontal assessment battery, Raven's Colored Progressive Matrices, and the Geriatric Depression Rating Scale. If necessary, dopamine transporter imaging and metaiodobenzylguanidine myocardial scintigraphy were used to diagnose DLB. Cerebrospinal fluid biomarkers and pathological examinations were not used for the diagnosis of dementia.

The AD patients in this study were probable AD. The diagnosis of AD and MCI was based on the criteria of the National Institute on Aging Alzheimer's Association workgroups (McKhann GM, Knopman DS, Chertkow H, Hyman BT, Jack CR, Jr., Kawas CH, Klunk WE, Koroshetz WJ, Manly JJ, Mayeux R et al: The diagnosis of dementia due to Alzheimer's disease: recommendations from the National Institute on Aging-Alzheimer's Association workgroups on diagnostic guidelines for Alzheimer's disease. Alzheimers Dement 2011,7(3):263-269;Albert MS,DeKosky ST,Dickson D, Dubois B, Feldman HH, Fox NC, Gamst A, Holtzman DM, Jagust WJ, Petersen RC et al: The diagnosis of mild cognitive disorder due to Alzheimer's disease: recommendations from the National Institute on Aging-Alzheimer's Association workgroups on diagnostic guidelines for Alzheimer's disease. Alzheimers Dement 2011, 7(3):270-279.). VaD and DLB subjects were respectively diagnosed according to the NINDS-AIREN International Workshop (Roman GC, Tatemichi TK, Erkinjuntti T, Cummings JL, Masdeu JC, Garcia JH, Amaducci L, Orgogozo JM, Brun A, Hofman A et al: Vascular dementia: diagnostic criteria for research studies. Report of the NINDS-AIREN International Workshop. Neurology 1993,43(2):250-260) and the fourth report of the DLB Consortium (McKeith IG, Boeve BF, Dickson DW, Halliday G, Taylor JP, Weintraub D, Aarsland D, Galvin J, Attems J, Ballard CG et al: Diagnosis and management of dementia with Lewy bodies: Fourth consensus report of the DLB Consortium. Neurology 2017, 89(1):88-100.). All healthy controls had MMSE scores >23.

All data in this study were obtained from publicly available sources.

## Detection of miRNA Expression Abundance

Serum miRNA extraction and expression profiling analysis are described in Shigemizu D, Akiyama S, Asanomi Y, Boroevich KA, Sharma A, Tsunoda T, Matsukuma K, Ichikawa M, Sudo H, Takizawa S et al: Risk prediction models for dementia constructed by supervised principal component analysis using miRNA expression data. Commun Biol 2019, 2:77. Briefly, serum samples were separated and transferred to a freezer at -80°C for storage. Total RNA was then extracted, and subjected to comprehensive miRNA expression analysis by the human miRNA Oligo array, which was designed to detect 2562 miRNA sequences. Normalization of miRNA expression was then performed.

## Screening of Differentially Expressed miRNAs

Firstly, the miRNA probes were ranked from high to low in terms of expression abundance, and the top 1000 miRNA probes were selected. To further identify differentially expressed miRNAs, the Limma package in R software was used. According to the criteria of $P < 0.05$ and fold change > 1.3, 22 miRNAs were finally obtained.

## Construction of System Model

Lasso logistic regression combined with 5-fold cross validation was used to build the model in the training set data, then validating in the validation set data. The optimal model was determined by scaling the negative log likelihood ($-\text{Log } L(\beta)$). The smaller the scaled $-\text{Log } L(\beta)$ value in the validation set, the better the model fit. Finally, 11 miRNAs, the subjects' apolipoprotein E4 genotype, the subjects' age, and the subjects' gender were included in the final model as independent variables (see Table 1). Table 1 shows the estimated parameter values, P values, and contributions of each variable in the model construction process.

Table 1: Parameter estimation results of the prediction model

| Variable | Estimated parameter value | Standard deviation | Wald $\chi^2$ | P value | Contribution (%) |
|---|---|---|---|---|---|
| Age | 0.185 | 0.018 | 100.493 | <.0001 * | 53.4 |
| Gender | -0.774 | 0.203 | 14.581 | 0.0001 * | 3.5 |
| APOE4 [homozygous vs No expression of☐APOE4] | 2.410 | 0.628 | 14.718 | 0.0001 * | 11.1 |
| APOE4 [heterozygous vs No expression of☐APOE4] | 1.188 | 0.242 | 24.091 | <.0001 * | |
| hsa-miR-6761-3p | 0.918 | 0.255 | 13.015 | 0.0003* | 11.0 |
| hsa-miR-3173-5p | -0.382 | 0.196 | 3.799 | 0.0513 | 2.9 |
| hsa-miR-6716-3p | 0.355 | 0.126 | 7.870 | 0.0050* | 2.6 |

(continued)

| Variable | Estimated parameter value | Standard deviation | Wald $\chi^2$ | P value | Contribution (%) |
|---|---|---|---|---|---|
| hsa-miR-6736-3p | 0.244 | 0.218 | 1.254 | 0.2629 | 0.8 |
| hsa-miR-6131 | -0.115 | 0.094 | 1.513 | 0.2187 | 0.8 |
| hsa-miR-6762-3p | 0.174 | 0.247 | 0.501 | 0.4792 | 0.5 |
| hsa-miR-6778-3p | -0.150 | 0.259 | 0.336 | 0.5620 | 0.3 |
| hsa-miR-6875-3p | 0.101 | 0.280 | 0.131 | 0.7171 | 0.1 |
| hsa-miR-6740-3p | 0.081 | 0.285 | 0.081 | 0.7762 | 0.1 |
| hsa-miR-24-3p | 0.029 | 0.126 | 0.053 | 0.8184 | 0.1 |
| hsa-miR-4747-3p | 0.001 | 0.160 | 0.000 | 0.9934 | 0.0 |

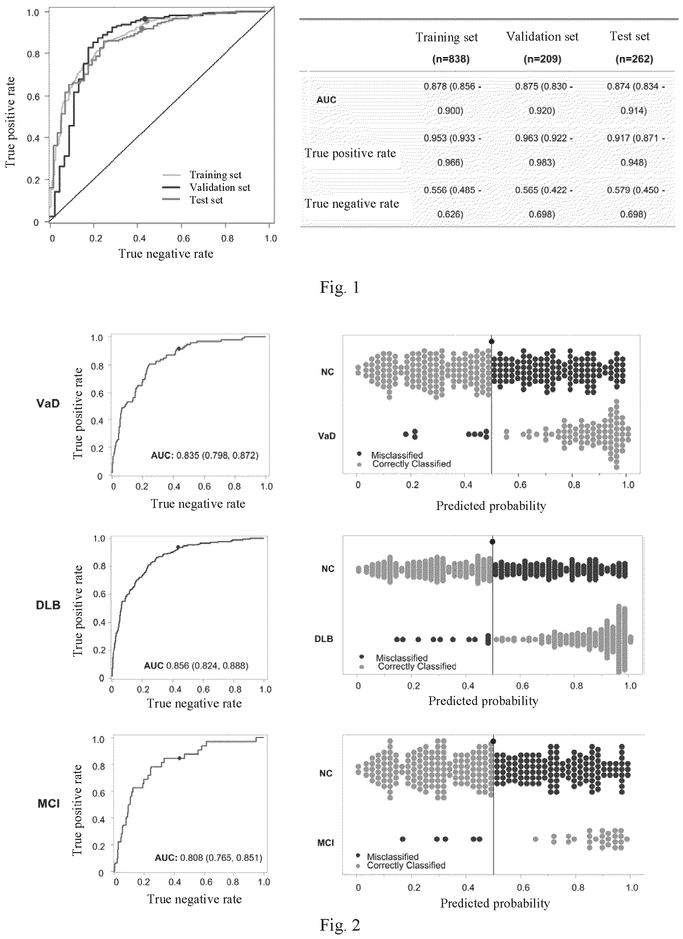

Table 1 shows that APOE4 allele status and female gender are the main risk factors for mild cognitive disorder and dementia, accounting for 53.4%, 11.1%, and 3.5%, respectively. Only three of the 11 miRNAs contributed the most, *namely,* hsa-miR-6761-3p, hsa-miR-3173-5p and hsa-miR-6716-3p. Therefore, the other 8 miRNAs with small contributions were deleted and re-modeled. The modeling results are shown in Fig. 1.

Based on the above method, the following Formula 1 was confirmed as the prediction model in this example:

$$p=1/[1+e^{-(i+a*Age+b*Gender+c*APOE\ Typing+d*hsa\text{-}miR\text{-}6761\text{-}3p+f*hsa\text{-}miR\text{-}3173\text{-}5p+g*hsa\text{-}miR\text{-}6716\text{-}3p)}]$$

(Formula 1)

wherein p is the probability of suffering from dementia or early cognitive disorder;
i is -19.23944, a is 0.2039311, d is 1.2992589, f is -0.398038, and g is 0.2794064;
when the subject is female, b is 0; when the subject is male, b is -0.726898;
when the subject does not express the APOE4 genotype, the c value is 0; when the subject's APOE4 is homozygous, the c value is 2.4565261; when the subject's APOE4 is heterozygous, the c value is 1.5060673.

The predicted effects of the model constructed by the above method on other dementia types and MCI is shown in Fig. 2.

The model established in this application by using AD data only comprises non-invasive parameters such as the levels of three miRNAs (miR-6761-3p, miR-3173-5p and miR-6716-3p) of the subject, the apolipoprotein E4 (APOE4) genotype of the subject, the age of the subject, and the gender of the subject. It performed well not only in predicting AD, but also in predicting VaD dementia, DLB dementia, and MCI, with AUCs of 0.874 (0.834, 0.914) in the AD validation data, 0.835 (0.798, 0.872) in VaD dementia, 0.856 (0.824, 0.888) in DLB dementia, and 0.808 (0.765, 0.851) in MCI, respectively.

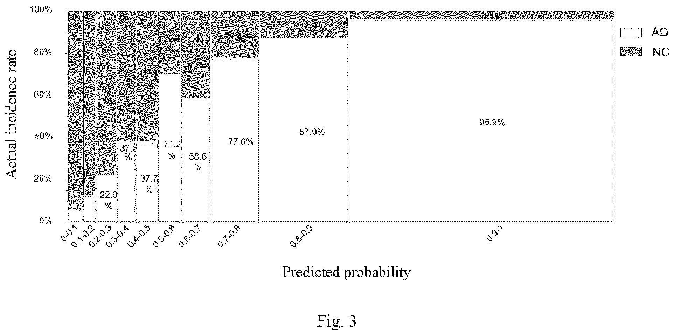

The grouping results of the constructed model in the AD validation set are shown in Fig. 3, wherein AD represents patients actually suffering from AD, and NC represents negative controls. From the results of Fig. 3, the following grouping basis of the model for predicting dementia or mild cognitive disorder can be obtained:
When the calculated probability (p) of the subject suffering from dementia or mild cognitive disorder is less than 10%, the risk of the subject suffering from dementia or mild cognitive disorder is low. As shown in Fig. 3, when the calculated p value is 0-0.1, the actual incidence of AD is about 5%, indicating that the risk of suffering from AD is at a low level.

When the calculated probability (p) of the subject suffering from dementia or mild cognitive disorder is equal to or greater than 10% and less than 50%, the risk of the subject suffering from dementia or mild cognitive disorder is medium. As shown in Fig. 3, when the calculated p value is 0.1-0.2, the actual incidence of AD does not exceed 10%, when the calculated p value is 0.2-0.3, the actual incidence of AD is about 20%, and when the calculated p value is 0.3-0.5, the actual incidence of AD is less than 30-40%, indicating that the subject has a certain risk of suffering from AD.

When the calculated probability (p) of the subject suffering from dementia or mild cognitive disorder is equal to or greater than 50% and less than 90%, the risk of the subject suffering from dementia or mild cognitive disorder is medium-high. As shown in Fig. 3, when the calculated p value is 0.5-0.9, the actual incidence of AD is higher than 50%, and in some cases it can be as high as 87%, indicating that the risk of suffering from dementia or mild cognitive disorder is already quite high.

When the calculated probability (p) of the subject suffering from dementia or mild cognitive disorder is equal to or greater than 90%, the risk of the subject suffering from dementia or mild cognitive disorder is high. As shown in Fig. 3, when the calculated p value is 0.9-1, the actual incidence of AD should exceed 95%, indicating that the risk of such subject is extremely high.

Fig. 3 shows the grouping verification based on AD patients. In actual situations, since AD has the largest sample size, it is the most representative. Therefore, the calculation results based on the above Fig. 3 basically confirm the grouping basis of the prediction model of the present application.

Although the embodiments of the present application are described above in conjunction with the accompanying drawings, the present application is not limited to the above particular embodiments and application fields. The above particular embodiments are merely illustrative and guiding, but not restrictive. Under the guidance of this specification and without departing from the scope of protection of the claims of the present application, a person skilled in the art can also make many forms, and they all fall in the protect scope of the present application.

**Claims**

1. A system for predicting dementia or mild cognitive disorder, comprising:

   a data acquisition module for acquiring data on miRNA level, apolipoprotein E4 genotype, age and gender of a subject; and
   a module for calculating the probability of suffering from dementia or mild cognitive disorder, which is used to calculate the data obtained in the data acquisition module, thereby determining the probability (p) of the subject suffering from dementia or mild cognitive disorder.

2. The system according to claim 1, wherein the miRNA is one or more selected from the group consisting of: hsa-miR-6761-3p, hsa-miR-3173-5p and hsa-miR-6716-3p; preferably the miRNA comprises hsa-miR-6761-3p, hsa-miR-3173-5p and hsa-miR-6716-3p.

3. The system according to claim 1, wherein the apolipoprotein E4 genotype of the subject refers to the typing of the apolipoprotein E4 allele.

4. The system according to claim 1, wherein in the module for calculating the probability of suffering from dementia or mild cognitive disorder, a formula for calculating the probability (p) of suffering from dementia is pre-stored, and the formula is obtained by fitting through logistic regression on the basis of the data on the miRNA level, apolipoprotein E4 genotype, age and gender of subjects in an existing database.

5. The system according to claim 4, wherein the formula is the following Formula 1:

$$p = 1/[1 + e^{-(i+a*Age+b*Gender+c*APOE\ Typing+d*hsa\text{-}miR\text{-}6761\text{-}3p+f*hsa\text{-}miR\text{-}3173\text{-}5p+g*hsa\text{-}miR\text{-}6716\text{-}3p)}]$$

(Formula 1);

   wherein p is the probability of suffering from dementia or mild cognitive disorder; APOE typing is the apolipoprotein E4 genotype of the subject, and i, a, b, c, d, f and g are unitless parameters;
   in the module for calculating the probability of suffering from dementia, the values of a, b, c, d, f and g are obtained on the basis of the miRNA level, apolipoprotein E4 genotype, age and gender of the subject, and the values are substituted into Formula 1 for calculation.

6. The system according to claim 5, wherein

   i is any value selected from -22.77226 to -15.70663, preferably -19.23944;
   a is any value selected from 0.1653123 to 0.2425499, preferably 0.2039311;
   d is any value selected from 0.8932064 to 1.7053113, preferably 1.2992589;
   f is any value selected from -0.722253 to -0.073824, preferably -0.398038; and
   g is any value selected from 0.0263939 to 0.5324189, preferably 0.2794064.

7. The system according to claim 5, wherein

   when the subject is a female, b takes the value of 0; and
   when the subject is a male, b is any value selected from -1.144378 to -0.309418, preferably -0.726898.

8. The system according to claim 5, wherein

when the subject does not express the APOE4 genotype, c takes the value of 0;
when the subject's APOE4 genotype is homozygous, c takes any value selected from 1.1429478 to 3.7701044, preferably 2.4565261; and
when the subject's APOE4 genotype is heterozygous, c takes any value selected from 0.9740697 to 2.038065, preferably 1.5060673.

9. The system according to claim 1, further comprising:
a grouping module, in which default grouping parameters for dementia or mild cognitive disorder are pre-stored, and the calculated probability (p) of the subject suffering from dementia or mild cognitive disorder is grouped on the basis of the grouping parameters, thereby grouping the risk of the subject suffering from dementia or mild cognitive disorder.

10. The system according to claim 9, wherein a grouping basis pre-stored in the grouping module is:

when the calculated probability (p) of the subject suffering from dementia or mild cognitive disorder is less than 10%, the risk of the subject suffering from dementia or mild cognitive disorder is low;
when the calculated probability (p) of the subject suffering from dementia or mild cognitive disorder is equal to or greater than 10% and less than 50%, the risk of the subject suffering from dementia or mild cognitive disorder is medium;
when the calculated probability (p) of the subject suffering from dementia or mild cognitive disorder is equal to or greater than 50% and less than 90%, the risk of the subject suffering from dementia or mild cognitive disorder is medium-high; and
when the calculated probability (p) of the subject suffering from dementia or mild cognitive disorder is equal to or greater than 90%, the risk of the subject suffering from dementia or mild cognitive disorder is high.

11. A method for predicting dementia or mild cognitive disorder, comprising:

a data acquisition step, which obtains data on miRNA level, apolipoprotein E4 genotype, age and gender of a subject; and
a step for calculating the probability of suffering from dementia or mild cognitive disorder, which is used to calculate the data acquired in the data acquisition step, thereby determining the probability (p) of the subject suffering from dementia or mild cognitive disorder.

12. The method according to claim 11, wherein the miRNA is one or more selected from the group consisting of: hsa-miR-6761-3p, hsa-miR-3173-5p and hsa-miR-6716-3p; preferably the miRNA comprises hsa-miR-6761-3p, hsa-miR-3173-5p and hsa-miR-6716-3p.

13. The method according to claim 11, wherein the apolipoprotein E4 genotype of the subject refers to the typing of the apolipoprotein E4 allele.

14. The method according to claim 11, wherein in the step for calculating the probability of suffering from dementia or mild cognitive disorder, a formula for calculating the probability (p) of suffering from dementia is pre-stored, and the formula is obtained by fitting through logistic regression on the basis of the data on the miRNA level, apolipoprotein E4 genotype, age and gender of subjects in an existing database.

15. The method according to claim 14, wherein the formula is the following Formula 1:

$$p = 1/[1 + e^{-(i + a*Age + b*Gender + c*APOE\ Typing + d*hsa\text{-}miR\text{-}6761\text{-}3p + f*hsa\text{-}miR\text{-}3173\text{-}5p + g*hsa\text{-}miR\text{-}6716\text{-}3p)}]$$

(Formula 1);

wherein p is the probability of suffering from dementia or mild cognitive disorder; APOE typing is the apolipoprotein E4 genotype of the subject, and i, a, b, c, d, f and g are unitless parameters;
in the step for calculating the probability of suffering from dementia, the values of a, b, c, d, f and g are obtained on the basis of the miRNA level, apolipoprotein E4 genotype, age and gender of the subject, and the values are substituted into Formula 1 for calculation.

**16.** The method according to claim 15, wherein

i is any value selected from -22.77226 to -15.70663, preferably -19.23944;
a is any value selected from 0.1653123 to 0.2425499, preferably 0.2039311;
d is any value selected from 0.8932064 to 1.7053113, preferably 1.2992589;
f is any value selected from -0.722253 to -0.073824, preferably -0.398038; and
g is any value selected from 0.0263939 to 0.5324189, preferably 0.2794064.

**17.** The method according to claim 15, wherein

when the subject is a female, b takes the value of 0; and
when the subject is a male, b is any value selected from -1.144378 to -0.309418, preferably -0.726898.

**18.** The method according to claim 15, wherein

when the subject does not express the APOE4 genotype, c takes the value of 0;
when the subject's APOE4 genotype is homozygous, c takes any value selected from 1.1429478 to 3.7701044, preferably 2.4565261; and
when the subject's APOE4 genotype is heterozygous, c takes any value selected from 0.9740697 to 2.038065, preferably 1.5060673.

**19.** The method according to claim 11, further comprising:
a grouping step, in which default grouping parameters for dementia or mild cognitive disorder are pre-stored, and the calculated probability (p) of the subject suffering from dementia or mild cognitive disorder is grouped on the basis of the grouping parameters, thereby grouping the risk of the subject suffering from dementia or mild cognitive disorder.

**20.** The method according to claim 19, wherein a grouping basis pre-stored in the grouping step is:

when the calculated probability (p) of the subject suffering from dementia or mild cognitive disorder is less than 10%, the risk of the subject suffering from dementia or mild cognitive disorder is low;
when the calculated probability (p) of the subject suffering from dementia or mild cognitive disorder (p) is equal to or greater than 10% and less than 50%, the risk of the subject suffering from dementia or mild cognitive disorder is medium;
when the calculated probability (p) of the subject suffering from dementia or mild cognitive disorder (p) is equal to or greater than 50% and less than 90%, the risk of the subject suffering from dementia or mild cognitive disorder is medium-high; and
when the calculated probability (p) of the subject suffering from dementia or mild cognitive disorder is equal to or greater than 90%, the risk of the subject suffering from dementia or mild cognitive disorder is high.

| | Training set (n=838) | Validation set (n=209) | Test set (n=262) |
|---|---|---|---|
| AUC | 0.878 (0.856 - 0.900) | 0.875 (0.830 - 0.920) | 0.874 (0.834 - 0.914) |
| True positive rate | 0.953 (0.933 - 0.966) | 0.963 (0.922 - 0.983) | 0.917 (0.871 - 0.948) |
| True negative rate | 0.556 (0.485 - 0.626) | 0.565 (0.422 - 0.698) | 0.579 (0.450 - 0.698) |

Fig. 1

Fig. 2

Fig. 3

## EP 4 524 991 A1

<table>
<tr><td colspan="2"><strong>INTERNATIONAL SEARCH REPORT</strong></td><td>International application No.<br><strong>PCT/CN2022/093822</strong></td></tr>
</table>

**A.  CLASSIFICATION OF SUBJECT MATTER**

G16H 50/30(2018.01)i;  G16H 50/50(2018.01)i;  G16H 50/70(2018.01)i;  G16B 40/00(2019.01)i

According to International Patent Classification (IPC) or to both national classification and IPC

**B.  FIELDS SEARCHED**

Minimum documentation searched (classification system followed by classification symbols)

G16H,  G16B

Documentation searched other than minimum documentation to the extent that such documents are included in the fields searched

Electronic data base consulted during the international search (name of data base and, where practicable, search terms used)

EPODOC, WPI, CNPAT, CNKI, IEEE: 阿尔兹海默症, 痴呆, 轻度认知障碍, 评估, 预测, 微小RNA, miRNA, 载脂蛋白E4, APOE4, 年龄, 性别, 男, 女, Alzheimer Disease, MCI, estimate, predict, age, gender, male, female

**C.  DOCUMENTS CONSIDERED TO BE RELEVANT**

| Category* | Citation of document, with indication, where appropriate, of the relevant passages | Relevant to claim No. |
|---|---|---|
| X | 廖峥奕 等 (LIAO, Zhengluan et al.). "基于血清小分子核糖核酸的阿尔茨海默病诊断模型构建与验证 (Construction and Validation of Diagnostic Models for Alzheimer's Disease based on Serum MicroRNAs)" <br> 中华精神科杂志 (Chinese Journal of Psychiatry), <br> Vol. 54, No. 5, 31 October 2021 (2021-10-31), <br> page 331, abstract, page 332, left-hand column, paragraph 2-page 335, right-hand column, paragraph 1, page 336, figure 5 | 1-20 |
| X | CHENG, L. et al. "Molecular Psychiatry" <br> Prognostic serum miRNA biomarkers associated with Alzheimer's disease shows concordance with neuropsychological and neuroimaging assessment, 28 October 2014 (2014-10-28), <br> page 1, abstract, page 2, left-hand column, paragraph 3-page 7, left-hand column, paragraph 1 | 1-20 |
| A | CN 108597615 A (NANJING BRAIN HOSPITAL) 28 September 2018 (2018-09-28) <br> entire document | 1-20 |
| A | CN 113383090 A (LA TROBE UNIVERSITY) 10 September 2021 (2021-09-10) <br> entire document | 1-20 |
| A | CN 110268269 A (SELONTERRA INC.) 20 September 2019 (2019-09-20) <br> entire document | 1-20 |

☑ Further documents are listed in the continuation of Box C.      ☑ See patent family annex.

| | |
|---|---|
| * Special categories of cited documents: <br> "A"  document defining the general state of the art which is not considered to be of particular relevance <br> "E"  earlier application or patent but published on or after the international filing date <br> "L"  document which may throw doubts on priority claim(s) or which is cited to establish the publication date of another citation or other special reason (as specified) <br> "O"  document referring to an oral disclosure, use, exhibition or other means <br> "P"  document published prior to the international filing date but later than the priority date claimed | "T"  later document published after the international filing date or priority date and not in conflict with the application but cited to understand the principle or theory underlying the invention <br> "X"  document of particular relevance; the claimed invention cannot be considered novel or cannot be considered to involve an inventive step when the document is taken alone <br> "Y"  document of particular relevance; the claimed invention cannot be considered to involve an inventive step when the document is combined with one or more other such documents, such combination being obvious to a person skilled in the art <br> "&"  document member of the same patent family |

| Date of the actual completion of the international search <br> **18 November 2022** | Date of mailing of the international search report <br> **25 November 2022** |
|---|---|
| Name and mailing address of the ISA/CN <br><br> **China National Intellectual Property Administration (ISA/CN)** <br> **No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2022/093822** |

| C. | DOCUMENTS CONSIDERED TO BE RELEVANT | |
|---|---|---|
| Category* | Citation of document, with indication, where appropriate, of the relevant passages | Relevant to claim No. |
| A | WO 2015179909 A1 (THE UNIVERSITY OF MELBOURNE et al.) 03 December 2015 (2015-12-03)<br>    entire document | 1-20 |

Form PCT/ISA/210 (second sheet) (January 2015)

**INTERNATIONAL SEARCH REPORT**
**Information on patent family members**

International application No.

**PCT/CN2022/093822**

| Patent document cited in search report | | | Publication date (day/month/year) | Patent family member(s) | | | Publication date (day/month/year) |
|---|---|---|---|---|---|---|---|
| CN | 108597615 | A | 28 September 2018 | None | | | |
| CN | 113383090 | A | 10 September 2021 | EP | 3891295 | A1 | 13 October 2021 |
| | | | | US | 2022017962 | A1 | 20 January 2022 |
| | | | | AU | 2019391602 | A1 | 24 June 2021 |
| | | | | WO | 2020113271 | A1 | 11 June 2020 |
| CN | 110268269 | A | 20 September 2019 | EP | 3555629 | A2 | 23 October 2019 |
| | | | | US | 2019338363 | A1 | 07 November 2019 |
| | | | | WO | 2018112446 | A2 | 21 June 2018 |
| | | | | HK | 40014761 | A0 | 21 August 2020 |
| WO | 2015179909 | A1 | 03 December 2015 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Commun Biol*, 2019, vol. 2, 77
- **MCKHANN GM ; KNOPMAN DS ; CHERTKOW H ; HYMAN BT ; JACK CR, JR. ; KAWAS CH ; KLUNK WE ; KOROSHETZ WJ ; MANLY JJ ; MAYEUX R et al.** The diagnosis of dementia due to Alzheimer's disease: recommendations from the National Institute on Aging-Alzheimer's Association workgroups on diagnostic guidelines for Alzheimer's disease. *Alzheimers Dement*, 2011, vol. 7 (3), 263-269
- **ALBERT MS ; DEKOSKY ST ; DICKSON D ; DUBOIS B ; FELDMAN HH ; FOX NC ; GAMST A ; HOLTZMAN DM ; JAGUST WJ ; PETERSEN RC et al.** The diagnosis of mild cognitive disorder due to Alzheimer's disease: recommendations from the National Institute on Aging-Alzheimer's Association workgroups on diagnostic guidelines for Alzheimer's disease. *Alzheimers Dement*, 2011, vol. 7 (3), 270-279
- **ROMAN GC ; TATEMICHI TK ; ERKINJUNTTI T ; CUMMINGS JL ; MASDEU JC ; GARCIA JH ; AMADUCCI L ; ORGOGOZO JM ; BRUN A ; HOFMAN A et al.** Vascular dementia: diagnostic criteria for research studies. Report of the NINDS-AIREN International Workshop. *Neurology*, 1993, vol. 43 (2), 250-260
- **MCKEITH IG ; BOEVE BF ; DICKSON DW ; HALLIDAY G ; TAYLOR JP ; WEINTRAUB D ; AARSLAND D ; GALVIN J ; ATTEMS J ; BALLARD CG et al.** Diagnosis and management of dementia with Lewy bodies: Fourth consensus report of the DLB Consortium. *Neurology*, 2017, vol. 89 (1), 88-100
- **SHIGEMIZU D ; AKIYAMA S ; ASANOMI Y ; BOROEVICH KA ; SHARMA A ; TSUNODA T ; MATSUKUMA K, ICHIKAWA M ; SUDO H ; TAKIZAWA S et al.** Risk prediction models for dementia constructed by supervised principal component analysis using miRNA expression data. *Commun Biol*, 2019, vol. 2, 77